# EUROPEAN PATENT SPECIFICATION

(11) **EP 2 094 310 B1**
(45) Date of publication and mention of the grant of the patent: **25.12.2013**
(21) Application number: 07846847.7
(22) Date of filing: 23.11.2007
(51) Int. Cl.: A61K 49/00

(54) **MARKER GENES FOR USE IN THE IDENTIFICATION OF CHONDROCYTE PHENOTYPIC STABILITY AND IN THE SCREENING OF FACTORS INFLUENCING CARTILAGE PRODUCTION**
MARKERGENE ZUR VERWENDUNG BEI DER IDENTIFIZIERUNG VON CHONDROZYTEN-PHÄNOTYPISCHER STABILITÄT UND BEIM SCREENEN VON FAKTOREN ZUR BEEINFLUSSUNG DER KNORPELPRODUKTION
GÈNES MARQUEURS SERVANT À IDENTIFIER LA STABILITÉ DE CHONDROCYTES PHENOTYPIQUES ET AU CRIBLAGE DE FACTEURS INFLUENCANT LA PRODUCTION DE CARTILAGE

(30) Priority: 24.11.2006 US 867152 P
(43) Date of publication of application: 02.09.2009
(73) Proprietor: TIGENIX N.V., 3001 Leuven (BE)
(72) Inventor: LUYTEN, Frank, 1950 Kraainem (BE); DE BARI, Cosimo, Aberdeen AB15 5HX (GB); DELL'ACCIO, Francesco, Bromley, Kent BR2 0BT (GB)
(74) Representative: De Baere, Ivo
(86) International application number: PCT/EP2007/010285
(87) International publication number: WO 2008/061804

(56) References cited:
- EP-A- 1 498 146
- WO-A-01/24833
- US-A1- 2002 009 730
- US-A1- 2003 235 813
- DELL'ACCIO F. ET AL.: "Molecular markers predictive of the capacity of expanded human articular chondrocytes to form stable cartilage in vivo" ARTHRITIS AND RHEUMATISM, LIPPINCOTT, PHILADELPHIA, US, vol. 44, no. 7, 1 July 2001 (2001-07-01), pages 1608-1619, XP002265720 ISSN: 0004-3591

### SEQUENCE LISTING

<110> Tigenix N.V.
   Luyten, Frank
   De Bari, Cosimo
   Dell'Accio, Francesco
<120> Marker genes for use in the identification of chondrocyte phenotypic stability and in the screening of factors influencing cartilage production
<130> T4503-PCT
<150> US 60/867,152
   <151> 2006-11-24
<160> 14
<170> PatentIn version 3.3
<210> 1
   <211> 30
   <212> DNA
   <213> artificial sequence
<220>
   <223> PCR primer
<400> 1
   tgacggggtc acccacactg tgcccatcta 30
<210> 2
   <211> 30
   <212> DNA
   <213> artificial sequence
<220>
   <223> PCR primer
<400> 2
   ctagaagcat ttgcggtgga cgatggaggg 30
<210> 3
   <211> 19
   <212> DNA
   <213> artificial sequence
<220>
   <223> PCR primer
<400> 3
   gaactccatc tctccctgc 19
<210> 4
   <211> 21
   <212> DNA
   <213> artificial sequence
<220>
   <223> PCR primer
<400> 4
   gagactggat ttcaaggcaa g 21
<210> 5
   <211> 20
   <212> DNA
   <213> artificial sequence
<220>
   <223> PCR primer
<400> 5
   ccctgagtgg aagagtggag 20
<210> 6
   <211> 20
   <212> DNA
   <213> artificial sequence
<220>
   <223> PCR primer
<400> 6
   gaggcgtgag gtcttctgtg 20
<210> 7
   <211> 20
   <212> DNA
   <213> artificial sequence
<220>
   <223> PCR primer
<400> 7
   ttcaaggggc agtgggtaac 20
<210> 8
   <211> 20
   <212> DNA
   <213> artificial sequence
<220>
   <223> PCR primer
<400> 8
   taaggtgata gtccagcggg 20
<210> 9
   <211> 20
   <212> DNA
   <213> artificial sequence
<220>
   <223> PCR primer
<400> 9
   tgtaagtctg tgtgcgagcg 20
<210> 10
   <211> 20
   <212> DNA
   <213> artificial sequence
<220>
   <223> PCR primer
<400> 10
   gatttagttg cgtgcttgcc 20
<210> 11
   <211> 21
   <212> DNA
   <213> artificial sequence
<220>
   <223> PCR primer
<400> 11
   cgacggaggc aggagaagca g 21
<210> 12
   <211> 21
   <212> DNA
   <213> artificial sequence
<220>
   <223> PCR primer
<400> 12
   tgaagtcgcg gtgggcaatg g 21
<210> 13
   <211> 20
   <212> DNA
   <213> artificial sequence
<220>
   <223> PCR primer
<400> 13
   gctgaaagac gatgccactg 20
<210> 14
   <211> 20
   <212> DNA
   <213> artificial sequence
<220>
   <223> PCR primer
<400> 14
   aggaccccaa aggaccagac 20

## Description

### FIELD OF THE INVENTION

The present invention relates to methods and tools for determining chondrocyte phenotypic stability and screening systems for identifying compounds of use in the treatment of cartilage defects and cartilage related diseases.

### BACKGROUND

Repair of cartilage defects is mainly achieved by surgical methods such as bone marrow stimulation techniques or by the implantation of cartilage forming cells (chondrocytes, (chondro-) progenitors and precursors thereof or stem cells which develop into cartilage). In view of this, the identification of factors capable of positively affecting *in vivo* cartilage formation is of interest. It is indeed desirable to identify surgical procedures, or therapeutic methods or compounds capable of positively affecting cartilage formation mediated by local cells present in or in the vicinity of the defect. In addition, it is of interest for cell-based therapies to identify factors or treatments which can positively affect the ability of isolated cell populations that have been expanded or passaged *in vitro* to produce stable cartilage *in vivo.*

Different assays have been described wherein the expression of genes involved in cartilage formation is used as a parameter for screening compounds. US2002061514 describes a method wherein a reporter gene is responsive to the transcription factor SOX9. SOX9 is a high-mobility-group (HMG) domain transcription factor that is expressed in chondrocytes, chondro-progenitors and other tissues and has been found to be essential for chondrocyte differentiation and cartilage formation. Other methods are based on the expression levels of individual genes which are involved in cartilage metabolism and osteochondral defects [e.g. osteoporosis related genes in WO02081745].

Screening methods have also been described which are based on the *in vitro* differentiation of cells into cartilage. However, the predictive value of such models has been found to be limited and these assays require large amounts of cells and are time consuming.

WO200466723 provides an *in vivo* model wherein the effect of compounds on bone and cartilage formation in zebrafish is evaluated *in vivo.*

One of the most reliable models for assessing the ability of cells to form stable cartilage is the intramuscular implantation of cells into nude mice followed by the histological evaluation of the generated implants. The use of this nude mouse model, which in itself is time-consuming and impractical for high-throughput screening, was avoided with the technology disclosed in WO0124833. This patent application describes the use of molecular markers, identified based on the nude mouse model for cartilage formation, to evaluate the chondrogenic potential of expanded or passaged cells for transplantation purposes. Similarly, a set of suitable markers for chondrocyte phenotypic stability are described in US2003235813. However, while the identification of these markers provided a basis for the identification of cell populations capable of producing stable hyaline cartilage *in vivo,* there remains a need for further improvement. Arrays for expression analysis are known for example from US 2002009730.

### SUMMARY OF THE INVENTION

The present invention is based on the novel concept of providing a specific marker profile which is indicative of cartilage formation in any given circumstance, i.e. whether native to the cells or induced, which can be considered as the target profile in the identification of factors capable of affecting cartilage formation.

Accordingly, the present invention provides a set of markers, which can be reliably used to predict the cartilage forming potential of a population of cells. The expression level of these markers can be used to predict whether a cell population, e.g. *in vitro* or *in vivo,* is able to produce cartilage *in vivo* upon implantation or upon stimulation. This is of interest for determining the therapeutic potential of a cell population. The set of markers also provides a reliable tool to determine the impact of a given treatment on the chondrocyte phenotypic stability of a population of cells prior to implantation. Furthermore disclosed herein are combined treatment regimes comprising the administration of stem cells or osteochondral cells in a joint and the administration of a medicament affecting the chondrogenic potential of the administered cell population.

The phenotypic stability of a cell population *in vitro* can be used in screening assays for the identification of factors capable of affecting chondrocyte phenotypic stability *in vitro* and/or *in vivo.* The invention provides a set of markers indicative of chondrocyte phenotypic stability, the expression of which is used to predict the effect *in vitro* or *in vivo* of compounds and/or conditions on the ability of a cell population to produce cartilage *in vivo.* The use of these markers in screening assay allows the screening of a large number of compounds and/or conditions on chondrocytes, without performing time consuming and laborious animal experiments.

In one aspect of the invention methods for determining the ability of a cell population to produce stable hyaline cartilage are provided. More particularly, methods are provided for determining *in vitro* the ability of a cell population to produce stable hyaline cartilage *in vivo*. In particular embodiments, methods are provided which comprise determining the expression by the cell population of a set of at least three marker genes comprising FRZB, ALK1 and one or more markers selected from the group consisting of PEDF, COL11, COL2, FGFR3, OPN, BMP-2 and RASF-PLA.

In particular embodiments of methods provided herein, the cell population is contacted with a compound or condition.

In particular embodiments, methods are provided which comprise the steps of contacting a population of cells with a compound or condition, and determining the expression level in the population of cells of a set of at least three marker genes comprising FRZB, ALK1 and one or more marker genes selected from the group consisting of PEDF, COL11, COL2, FGFR3, OPN, BMP-2 and RASF-PLA. In these methods, the expression level of the set of at least three markers is indicative of the ability of the cells to produce stable hyaline cartilage in vivo, e.g. upon implantation into a patient.

Further embodiments of methods described herein comprise the steps of, prior to contacting the cell population with a compound or condition, determining in the population of cells the expression level of the set of at least three marker genes, and, after the contacting step, determining whether the the presence of the compound or condition affects the expression in the cell population of one or more of the set of at least three marker genes. More particularly, methods are provided which comprise determining, based on the effect of the presence of the compound or condition on the expression of the set of at least three marker genes in the cell population, the effect of the compound or condition on the ability of the cell population to produce stable hyaline cartilage *in vivo.* In particular embodiments of the methods described herein, compounds or conditions capable of increasing the expression of one or more positive marker genes selected from the group consisting of FRZB, COL11, COL2, FGFR3, OPN, BMP-2 and RASF-PLA are identified as positively affecting cartilage formation and compounds capable of increasing the expression of either PEDF and/or ALK-1 are identified as negatively affecting cartilage formation, more particularly the ability of cells to produce stable hyaline cartilage *in vivo.*

In further particular embodiments of methods described herein, the set of markers are a set of at least 4, 5 or 6 marker genes comprising FRZB, ALK1 and comprising respectively 2, 3 and 4 marker genes selected from the group consisting of PEDF, COL11, COL2, and FGFR3. More particular embodiments of methods described herein, relate to methods wherein the of marker genes is a set of at least six marker genes comprising FRZB, ALK1, PEDF, COL11, COL2 and FGFR3.

In particular embodiments of methods described herein, the ability of a compound or condition to affect cartilage formation is determined based on the cumulative effect of the compound or condition on the expression of the set of at least three marker genes. More particularly, the cumulative effect of the compound or condition on the expression of the positive marker genes selected from the group consisting of FRZB, COL11, COL2, FGFR3, OPN, BMP-2, and RASF-PLA and the cumulative effect of the compound or condition on the expression of negative marker genes PEDF and/or ALK-1 is indicative of the ability of the compound or condition to affect chondrocyte phenotypic stability of a cell population.

In particular embodiments of methods described herein, the population of cells is obtained from a joint. The population of cells is obtained from a healthy donor or from an individual with an osteochondral defect. In particular assays the effect of a compound or condition on both types of cells is envisaged. In particular embodiments of methods described herein the expression level of one or more of the markers is determined with a quantitative method.

A further aspect of the invention relates to the use of a set of markers for identifying a compound that is capable of affecting the ability of cells, more particularly chondrocytes or chondrocyte precursor cells, to produce cartilage *in vivo.* In particular embodiments the use is characterised in that the set of markers comprises a set of at least three marker genes comprising FRZB, ALK1 and one or more markers selected from the group consisting of PEDF, COL11, COL2, FGFR3, OPN, BMP-2 and RASF-PLA. In specific embodiments, the use involves a set of at least six marker genes comprising FRZB, ALK1, PEDF, COL11, COL2, and FGFR3.

A further aspect of the invention provides kits comprising probes for detecting the expression of a set of genes in cartilage forming cells wherein the set of genes consists of FRZB, ALK1, PEDF, COL11, COL2, and FGFR3.

Particular embodiments of the kits provided herein are kits wherein the probes are oligonucleotides which hybridise with mRNA, wherein the probes are antibodies and/or wherein the probes are sets of PCR primers specific for the markers to be tested. Kits are envisaged which comprise different types of probes. In further particular embodiments of the kits, labelled probes are provided.

Yet a further aspect of the invention provides devices for detecting the expression of a set of six marker genes consisting of FRZB, ALK1, PEDF, COL11, COL2, and FGFR3. in cells, more particularly cells typically capable of cartilage formation. In particular embodiments of such devices, the following components are provided: a unit for detecting the expression of marker genes, and probes for the above set of six markers.

### DETAILED DESCRIPTION OF THE INVENTION

### Definitions

The term "chondrogenic" when applied to a cell or a population refers to the inherent capacity of that cell or population to produce cartilage or to stimulate cartilage growth, under appropriate circumstances.

A "chondrogenic compound" as used herein refers to a compound which induces a stable chondrocyte phenotype and/or promotes cartilage formation.

The term "chondrocyte phenotypic stability" or "chondrogenic potential" when referring to a cell population, refers to the ability of the cell population to produce cartilage *in vivo.* This ability can be tested by (ectopically) injecting a fraction of the cell population (at least about 1 - 20 x 10⁶ cells) in a mammal (*in vivo*), such as immune-deficient mice, and determining (in a time frame of about 3 weeks), the development of a cartilage implant without signs of vascular invasion, mineralisation or replacement by bone or fibrous tissue. Alternatively, a fraction of the cell population (at least about 1 - 20 x 10⁶ cells per cm²) can be seeded onto, or encapsulated in, a biocompatible matrix and implanted subcutaneously under the skin for about 6-8 weeks before determining the development of a stable cartilage implant without signs of vascular invasion or endochondral bone formation. A population of cells that is capable of producing stable hyaline cartilage in vivo, is characterized by the presence of a specific combination of markers of phenotypic stability described herein. Chondrocyte phenotypic stability is gradually lost in mature chondrocytes upon passaging.

The term "freshly isolated cells" (FI) as used herein refers to the cell population obtained from a biopsy after tissue digestion. The term "freshly isolated cartilage cells" as used herein thus refers to the cell population directly obtained from a chondrocyte-containing tissue, such as cartilage, by digestion, without passaging.

The term "expanded", when referring to a cell population obtained from a tissue, such as cartilage, indicates that the cell population has been placed under conditions whereby the number of cells has increased by proliferation. In its simplest version, expansion is performed by providing the cell population in a cultivation recipient with appropriate cultivation medium, optionally until confluency. The population which is obtained as a result of expanding freshly isolated cells is referred to as P0.

The term "passaged" refers to cells which have been expanded, and which following expansion have been harvested from the cultivation recipient using enzymatic, chemical and/or physical methods and are placed at a lower density in another cultivation recipient. Passaged cells are referred to by their passage number (P1, P2, etc.) their time in culture or alternatively, by the number of their population doublings (PD1, PD2 etc).

The term "cartilage defect" as used herein refers to any condition resulting from the loss or damage of cartilage or any diseased portion of cartilage.

The term "marker score" as such is used to refer to a numeric value which can be attributed to the expression of a marker. The 'cumulative marker score' refers to the combination of the marker score of the different markers of the sets of markers. This can be the result of a sum of the individual markers. Alternatively, the cumulative marker score takes into account differences in the contribution of each marker in the ability to predict chondrocyte phenotypic stability and accordingly is based on a more complex mathematical formula.

The term "histology score" refers to a qualitative or semi-qualitative value attributed to cartilage based on a histological analysis (e.g. no cartilage, fibrocartilage, hyaline cartilage).

The abbreviations used for genes in the context of the present application are:

| | |
|---|---|
| **FRZB:** | Frizzled-Related Protein 1 [OMIM 605083, Genbank U91903] |
| **ALK1:** | Activin A Receptor, Type II-Like Kinase 1 [OMIM 601284, Genbank Z22533] |
| **PEDF:** | Pigment Epithelium-Derived Factor [OMIM 172860, Genbank M76979] |
| **COL11**: | Collagen, Type XI A1 [OMIM 120280, Genbank J04177] |
| **COL2:** | Collagen, Type II, Alpha 1 [OMIM 120140, Genbank L10347] |
| **FGFR3:** | Fibroblast Growth Factor Receptor 3 [OMIM 134934, Genbank M58051] |
| **BMP-2:** | Bone Morphogenetic Protein 2 [OMIM 112261, Genbank M22489] |
| **RASF-PLA2:** | Phospholipase A2, Group IIA [OMIM 172411, Genbank M22430] |
| **OPN:** | Osteopontin [OMIM 166490, Genbank X13694] |

The present invention is based on the identification of a selected set of marker genes, the expression of which has been found to be indicative for the ability cartilage formation of cells *in vivo.* More specifically, it has been determined that by attributing a value to each of these markers, the cumulative value, which can be expressed as a cumulative marker score, reflects the ability of a cell population to produce phenotypically stable cartilage *in vivo.*

The marker genes identified in the present invention as useful in the assessment of the ability of a cell population to ensure cartilage formation are selected from the group including the positive marker genes FRZB, COL11, COL2, FGFR3, BMP-2, RASF-PLA2, and OPN and the negative marker genes ALK1 and PEDF. Additional markers may be included in the set, e.g. as controls.

Thus, according to the present invention, a set of 9 markers has been identified, comprising both positive and negative markers. Positive markers are strongly expressed by populations capable of producing stable hyaline cartilage *in vivo*, while negative markers are expressed at low levels or not expressed by populations capable of producing stable hyaline cartilage. According to the present invention, the absence of expression of a negative marker, can serve as a positive marker. However, in the context of the present invention such markers of which the absence of expression is indicative of the ability of chondrocyte phenotypic stability, are referred to herein as negative markers.

Expression of marker genes is a feature characteristic of a cell population, typically consisting of around 0.2x10⁵ -1.0x10⁶ cells.

Different methods for determining expression of genes are known in the art. Most typically, according to the present invention, RNA is isolated from a fraction of the cell population (typically between 0.2 x10⁵ - 1.0x10⁶ cells and preferably between 0.2x10⁶ - 1x106) and amplified using reverse PCR. Such amplification can be performed in a semi-quantitative way via the electrophoresis of amplified fragments and measurement of their relative intensity compared with that of a housekeeping gene such as beta-actin. However, lower amounts of cells and even individual cells can be analysed for expression of cells using nucleotide amplification techniques. Additionally or alternatively, the amount of DNA is measured in a quantitative way using e.g. the Taqman technology. Primers which are suitable for the markers mentioned in this application can easily be identified by the skilled person based on the known sequences of the marker genes.

Alternatively or additionally, the expression of markers is measured at the protein level. Protein levels can be determined by immunological methods such as Western blots or ELISA.

In specific embodiments of the invention, the expression of markers is evaluated by comparing the expression of markers in a control population and an experimental population, such as via micro array technology or analysis after 10 or 2D protein electrophoresis. Herein, the marker proteins migrate in a gel in accordance with their Mr (1 D) and their iso-electric point (IEP) (2D). Differences in expression at the protein level can be measured by detecting the amount of protein present at the position corresponding with its Mr and/or IEP. Proteins can be quantified after staining with Coomassie Brilliant Blue or an equivalent staining or can be quantified by incorporating a radioactive metabolic label (eg ³⁵S methionine).

Depending on the technology used, antibodies, oligonucleotide probes or protein probes (such as antibodies) can be labelled, e.g. with chromophoric or magnetic or radioactive labels, so as to allow detection of expression.

In specific embodiments it is envisaged to detect different markers by different techniques. For example, a number of markers are detected by ELISA while others are detected via PCR.

A further aspect of the invention provides a device suitable for the detection of of a set of six marker genes comprising FRZB, ALK1, PEDF, COL11, COL2 and FGFR3, which device comprises a unit for directly or indirectly detecting the markers of the present invention (at DNA, RNA, or protein level). The unit can be based on RNA, DNA hybridization or based on immunological detection. Such a unit can be a (quantitative) PCR apparatus, a device for performing immunological assays, or a device for determining proteins (e.g. HPLC, mass spectrometer, electrophoresis apparatus), but can also be a (disposable) micro array device or chip. Such unit is, when applicable, equipped with probes or antibodies for detecting marker genes or proteins. Optionally, such a device can further comprise a cell-cultivation unit, wherein two or more cell populations can be grown under identical or under different conditions (e.g. density, passage number, medium composition). Furthermore the device of the invention can comprise a unit for delivering one or more test compounds to the cell-cultivation unit (which delivery can be individually varied in amount or in time-profile). Finally, the device can comprise a unit for collecting cells and isolating cellular material (DNA, RNA, proteins).

In addition, disposable cartridges are envisaged comprising probes or specific reagents for the markers described herein which can be used to determine the expression of these markers for a rapid and efficient determination of the status of a cell population. Accordingly, the present invention envisages automated devices, which are optionally combined with disposable cartridges, which allow efficient quality control of cells for use in *in vivo* or *in vitro* applications.

Subsets of the marker genes identified are provided which allow a reliable assessment of the chondrocyte phenotypic stability of a cell population or of the ability of a compound or condition to influence cartilage formation by a cell population *in vivo.* Most particularly, suitable sets of marker genes comprise at least 3, more particularly at least 4, even more particularly at least 5, and most particularly at least 6 of the identified marker genes.

In general, the lower the number of marker genes taken into account, the higher the impact of both measuring errors and the potential aberrant expression of one particular marker. The different markers also reflect the different processes (e.g. anabolic processes, catabolic processes, etc.) and pathways involved in chondrocyte cell metabolism (signalling pathways, receptors and ligands, matrix components, processing enzymes, ...) and each of these processes can have an individual impact on chondrocyte phenotypic stability. Thus, the more marker genes are taken into account, the more representative the assay becomes of the complete environment ensuring stable cartilage formation. On the other hand, the more marker genes are to be determined, the more technically complicated the assay becomes, compromising its ease of use in high-throughput screening. Also, in order to maintain the relevance of the contribution of each of the marker genes to the overall evaluation, the number of marker genes should be kept below 10. Indeed, the change of the expression of a single marker gene within a large set of marker genes will be of little influence on the overall expression score of the marker genes. Based on these arguments and the observed relative contribution of the different markers identified, it has been determined that any number of markers between 3 and 9 is suitable to determine the chondrocyte phenotypic stability of a cell population or to assess the influence of a compound or condition on chondrocyte phenotypic stability of a cell population. According to one embodiment of the methods and uses of the present invention, a set of 5, 6 or 7 markers is an optimal compromise between the accuracy of the obtained score from such a set of markers and the practical feasibility of the assay in high throughput screening.

Herein described is a set of at least three markers representative of cartilage formation selected from the group consisting of the positive marker genes FRZB, COL11, COL2, FGFR3, BMP-2, RASF-PLA2, and OPN and the negative marker genes ALK1 and PEDF.

The set of at least three markers making up the chondrogenic potential markers, i.e. representative of cartilage formation can include FRZB, ALK1 and a marker gene selected from the group consisting of PEDF, COL11, COL2, FGFR3, BMP-2, RASF-PLA2 and OPN. More particularly, the set of at least three markers representative of cartilage formation includes FRZB, ALK1 and a marker gene selected from the group consisting of PEDF, COL11, COL2, and FGFR3. Disclosed herein are sets of at least 4, 5 or 6 marker genes comprising FRZB, ALK1 and respectively 2, 3 and 4 markers selected from the group consisting of PEDF, COL11, COL2, FGFR3, BMP-2, RASF-PLA2 and OPN. More particularly, the sets of at least 4, 5 or 6 marker genes comprise FRZB, ALK1 and respectively 2, 3 and 4 markers selected from the group consisting of PEDF, COL11, COL2 and FGFR3. According to a particular embodiment, a set of six marker genes is used to asses the ability to produce stable cartilage *in vivo*, the set consisting of FRZB, ALK1, PEDF, COL11, COL2, and FGFR3. The cumulative score of this set of marker genes described in Example 1 referred to herein as the "ChondroCelect™ markers" (CC markers) is also referred to as the "ChondroCelect™ score". Alternatively, the set of markers comprises a variation of this set of chondrogenic potential markers wherein one of the markers PEDF, COL11, COL2 and FGFR3 is replaced by one marker selected from the group of RASF-PLA2, OPN and BMP-2. More particularly, the set of markers comprises a variation of the above-described chondrogenic potential markers, wherein one of the positive markers COL11, COL2 is replaced by either RASF-PLA, OPN or BMP-2. The examples section herein demonstrates that marker scores based on the expression of the sets of markers disclosed herein by a chondrogenic cell population reliably predict the nature of the cartilage produced by the cell population (as determined by histological analysis).

According to another aspect, the present invention provides methods of determining chondrocyte phenotypic stability of a cell population. More particularly, the present invention provides for the use of the sets of marker genes disclosed herein as a tool in the assessment of chondrocyte phenotypic stability of a cell population. This is of importance e.g. in the quality control of cell populations used in the transplantation of cells in the context of the treatment of cartilage defects. These methods are characterized in that they comprise the step of determining the expression by the cell population of a set of marker genes as described herein. More particularly, the expression of a set of marker genes according to the present invention, optionally expressed as a cumulative marker score, more particularly the Chondrogenic potential score, is used as an indication of the quality of a cell population for implantation purposes. Typically, the quality assessment of a cell population is performed in a laboratory using standard techniques such as those described herein and the outcome of the assessment is generated in a report or summarized in a quality control sheet. The cell population may be P0 or any passage of a cell population obtained from a human or animal tissue. According to one embodiment, the cell population is between P2 and P6 of a population obtained from a human cartilage biopsy. Alternatively, the number of population doublings can be considered. For most cell populations considered in the context of the present invention, every passage involves 2-3 population doublings. Accordingly in the context of the present invention, the cell population is assessed after 1 or two population doublings (P0). Optionally, the cell population is a combination of different passages (optionally including P0) of cells from one or from different biopsies. Where autologous transplantation of cells is intended, the cells obtained from one or more biopsies from a specific patient are optionally combined and checked for chondrocyte phenotypic stability (before and/or after combination thereof). Typically, the quality assessment is performed at specific time points, i.e. prior to implantation (as cells or 2D or 3D tissues in a scaffold or matrix), prior to storage (e.g. freezing) and/or retrieval from storage, prior to and/or after subjecting the cell population to specific treatments and/or conditions.

Indeed, the object of one aspect of the invention is to determine the ability of a cell population to produce stable hyaline cartilage in vivo prior to either direct implantation, or seeding onto, or encapsulation in a biocompatible matrix and subsequent implantation. The marker score is indicative of the ability of the cell population or cells to ensure a cartilage implant without signs of vascular invasion or endochondral bone formation.

Typically, the expression of the markers for determining the marker score according to the present invention is performed on a cell population as a whole, whereby a representative sample is taken to determine, by methods such as those described herein, whether or not the markers are expressed by the cell population under investigation. Alternatively, individual cells can be checked for the expression of specific genes using cell-specific methods known in the art.

According to another aspect, the invention provides methods for determining the effect of a compound or condition on the ability of chondrogenic cells to produce stable hyaline cartilage *in vivo,* which methods are of use as screening tools. These methods comprise the step of contacting a chondrogenic cell population with a compound or condition and, determining the expression of a set of marker genes according to the present invention, by that cell population.

Accordingly, methods and assays are provided for identifying compounds or conditions that affect the chondrogenic capacity of a population of cells. According to one embodiment, the method is used to identify adequate conditions for cultivating, storing and/or administering cell populations for transplantation in the context of the treatment of cartilage defects. In such methods, a chondrogenic cell population is subjected to specific conditions and the expression of the marker genes is determined. Typical conditions are specific cultivation media, cultivation temperatures, cultivation times, cultivation density etc.., as well as combinations with other cell types. Additionally or alternatively, the methods and assays are used for the identification of molecular factors capable of affecting chondrocyte phenotypic stability of chondrogenic cells. Such factors can include growth factors, mitogens, additives, small chemical molecules etc... The object of the screening can be to identify factors useful in the cultivation, storing or administration of cell populations for transplantation in the context of the treatment of cartilage defects. Typically, factors of interest are factors which are capable of positively affecting the chondrocyte phenotypic stability of a cell population and/or factors which positively affect other features of the cells (e.g., cell number, stability, etc..) and/or parameters of the cultivation conditions (e.g., storage time, number of passages that can be used, etc..) without negatively affecting the chondrocyte phenotypic stability of the cell population.

Additionally or alternatively, the assays and methods of the present invention are of use in the screening and identification of compounds with a potential effect *in vivo* on cartilage formation by chondrogenic cells. The use of the screening methods is envisaged for the identification of compounds which affect cartilage formation by local cells in the body of the patient to be treated and/or cartilage formation by cells used in the context of cell transplantation. The screening methods can be used to identify and/or evaluate compounds which can counteract factors or conditions occurring in pathologicail situations. For instance, cells can be stimulated e.g. with IL-1 (to stimulate inflammatory conditions, to enhance catabolic pathways and to downregulate anabolic processes) before addition of compounds that potentially counteract this effect.

Also envisaged is the use of the marker genes for assessing the potential side effects of therapeutic compounds, including those used outside the context of cartilage repair, on the ability of chondrogenic cells to produce cartilage *in vivo.* The methods of the present invention potentially allow the direct identification of effects of compounds on bone formation, which, when tested directly *in vivo,* would only be noticed on a longer term.

The methods relating to the screening of compounds, will typically comprise the step of contacting a chondrogenic cell population with one or more compounds of interest and determining the expression of a set of marker genes as described herein by the chondrogenic cell population. These methods allow high-throughput screening of e.g. chemical compound libraries, peptide libraries, expression libraries etc...

Methods are disclosed which comprise the step of comparing the expression of a set of marker genes in a cell population to which one or more compounds has been added/which has been subjected to a particular condition with the expression of the set of marker genes in the absence of the compound or condition in the same or a different cell population. In a typical embodiment, (a) different fraction(s) originating from the same cell populations are subjected to different conditions and/or compounds for comparison with each other and/or with a positive and/or negative control. The negative control can be a blank or a compound known not to affect chondrocyte phenotypic stability. The positive control can be a compound or condition known to (positively or negatively) affect the phenotypic stability of a cell population.

In screening methods, different parameters can be evaluated. Cells can be seeded in individual recipients or multiwell plates at different densities. Cells can be incubated during different time periods before adding a compound/factor or applying a condition or both. Different regimes and combinations of compounds and cultivation conditions can be combined. Compounds/factors can be added at different concentrations and remain in contact with the cells for different periods of time. The same applies to cultivation conditions.

The present invention is based on the observation that the ability of a cell population to produce hyaline cartilage *in vivo* after implantation can be predicted based on the expression of specific genes (the marker genes) by that cell population *in vitro,* prior to implantation. Thus, the expression of the marker genes is used to monitor the chondrogenic "quality" of the cells prior to implantation and the effects of compounds and conditions thereon. As detailed below, the expression of the genes can be expressed as a marker score, whereby the (relative) expression level of the different marker genes is added up to obtain a value, which can be compared to the optimal value (i.e. high expression of all positive markers, low expression of all negative markers under consideration). Additionally or alternatively, the screening can take into account the ability of the compounds or conditions to influence any one marker within this set of genes.

Expression of marker genes can be assessed based on absolute or relative value. Typically, the expression of the marker gene compared to a house-keeping gene is used. This is exemplified with actin herein. As the expression levels of different housekeeping genes may be different, the skilled person will understand that the relative expression of the marker genes compared to the selected housekeeping gene will need to be established beforehand in a population with chondrocyte phenotypic stability for each house-keeping gene used. According to the present invention, the combined information on the expression of the selected markers is indicative of whether or not the cells are capable of cartilage formation in vivo.

The present invention envisages the use of the marker combinations described herein both in the context of assessing the quality of a given cell population and for the evaluation of the influence of factors/conditions on the ability of cells to produce cartilage after implantation in vivo. Where the set of markers is used to determine the effect of a compound, cell or condition on the ability of a cell population to produce hyaline cartilage *in vivo,* the effect can be based on determining 'increased' or 'decreased' expression of the marker genes of the invention relative to a control or relative to the expression level prior to contacting the cell population with the compound or condition. In this regard, compounds, cells or conditions which increase expression of one or more positive marker genes and/or decrease the expression of one or more negative marker genes can be considered capable of positively affecting cartilage formation. Compounds, cells or conditions which decrease expression of one or more positive marker genes and/or increase the expression of one or more negative marker genes can be considered capable of negatively affecting cartilage formation.

The expression of the different marker genes of the set of markers by a cell population is represented by a 'cumulative marker score', which correlates with and is indicative of the chondrocyte phenotypic stability of the cell population. The cumulative marker score is used for the qualitative and/or quantitative interpretation of the results in the methods and assays of the present invention.

Different types of marker scores are envisaged within the context of the present invention. The expression level of a set of markers can be represented by a numerical value which is/corresponds to the sum of the absolute expression level of each of the gene products in the marker set. Such calculation method can be used when a quantitative detection of the expression of the marker genes is performed e.g. by quantitative PCR or ELISA.

Alternatively, the expression level of each of the genes in the marker set can be represented by a ratio, i.e. the expression level of a gene is compared to that of another gene such as a housekeeping gene which is considered to have an equal expression level regardless of the treatment of a cell or of which the expression is considered as relatively stable, independent of the ability of the cell to produce stable hyaline cartilage. This will result in the values of expression levels for the different markers being more comparable (and mostly within the 0.001 to 10 range). Accordingly, the cumulative marker score can be the sum of the ratios for each marker gene.

In certain conditions, however, the cumulative marker score is not based on the absolute or relative values of expression for each of the marker genes, but on the qualitative assessment of this expression.

According to a specific method, qualitative assessment of the marker genes is arbitrarily assigned a numerical value, e.g. '1'. More particularly, increased/high expression of the positive markers is assigned the value '1' and absence of expression of a negative marker is also assigned the value '1'. Most particularly, ranges of expression levels (or ratios of expression) are defined for both expression levels of positive marker genes and expression levels of negative markers of chondrocyte phenotypic stability, whereby expression levels falling within the predetermined ranges are attributed a value. According to a further embodiment, expression values falling outside the predetermined ranges of expression of the positive and/or negative marker genes (e.g. when very low expression of a positive marker gene is observed or very high expression of a negative marker gene) is also attributed a value.

When determining the relative impact of each of the marker genes in the cumulative marker score, the expression of each gene can have the same weight. Alternatively, the expression level of one or more genes can be considered as having a stronger impact on chondrocyte phenotypic stability than other genes, which can be incorporated in the relative impact of the marker genes on the calculation of the cumulative marker score., In a particular method the relative impact of the different markers is the same and is attributed the value '1' when falling within the predetermined ranges of expression (or of expression ratios) attributed the value '-1', when falling outside of the ranges (below or higher than the predetermined ranges for the positive and negative markers, respectively). Optionally, the expression levels within a certain range can be considered to correspond to a value '0', which corresponds to an expression level of the positive or negative marker gene which does not positively or negatively affect chondrocyte phenotypic stability in the cell.

In the examples section described herein, a further particular embodiment of the invention is described. The cumulative marker score representative of a stable cartilage phenotype is determined by a set of up to six markers, each of which is attributed equal importance. The expression of each of the markers is quantified, and a scoring approach based on expression relative to a marker gene is applied. In this specific embodiment, the score calculated from these markers is referred to as the "Chondrogenic potential score" i.e. a cumulative marker score which reflects the overall expression of a defined set of six markers of cartilage phenotypic stability According to this embodiment of the invention, the expression level of each gene is determined via a semi-quantitative method and the obtained values are normalised by comparison with a reference gene (e.g. beta actin). Thus, an expression level of 1 refers to an expression that is the same as that of actin, an expression level below 1 refers to an expression that is lower than that of actin (0.1 corresponding to an expression level which is 10x lower than that of beta-actin) and an expression level above 1 refers to an expression level that is higher than that of actin.

In this specific embodiment of the present invention, the expression level of each of the marker gene corresponds to one of three possible scores. In the case of a positive marker (FRZ, COL11, COL2, FGFR3) a very low expression level (0-0.1) is represented by a score of -1, a low expression level (0.1-1) is represented by a score of 0 and a high expression (>1) is represented by a score of +1. Inversely, for a negative marker (ALK1, PEDF) a very low expression level (0-0.1) is represented by a score of +1, a low expression level (0.1-1) is represented by a score of 0 and a high expression level (>1) is represented by a score of -1. The sum of all these individual scores represents the expression level of the entire set of markers as a whole. For a set of 6 markers, the score can thus range from -6 (all positive markers expressed at a level which is lower than the reference and all negative markers expressed at a level which is higher that the reference gene) to +6 (all positive markers expressed at a level which is higher than the reference gene and all negative markers expressed at a level which is lower than the reference gene). Where this score is based on the expression of the markers FRZB, COL11, COL2, FGFR3, ALK1 and PEDF by a cell population, this score is referred to herein as the Chondrocelect™ score.

As shown in the examples of the present invention, is it possible to correlate the expression levels of a set of markers, as well as the cumulative marker score obtained therefrom, with the chondrocyte phenotypic stability of a chondrocyte cell population. Accordingly, evaluation of the expression levels of a defined set of markers, or of the (cumulative) marker score of a chondrogenic cell population upon administration of certain compounds thereto, can be used to evaluate the ability of the compound to affect chondrocyte phenotypic stability of cells and/or the ability of cells to form cartilage *in vivo.* More particularly it is demonstrated that addition of particular compounds to an expanded chondrocyte cell population improves the Chondrogenic potential score and the phenotype of this cell population. Negative controls, e.g. addition of compounds which promote bone formation decrease the Chondrogenic potential score.

Accordingly, cumulative marker scores are provided which can be used in a cell-based screening assay to assess the influence of any factor or condition on the capacity of a chondrogenic population to produce stable cartilage. In view of the fact that the cartilage phenotypic stability of a cell population as identified *in vitro,* is representative of the activity of the cells *in vivo,* the assay can be reliably used to identify compounds capable of influencing cartilage formation by local chondrogenic cells upon administration to the cartilage defect. Importantly, the assay and marker analysis as disclosed herein do not require a subsequent or parallel *in vitro* or *in vivo* cartilage formation assay to validate the results, which dramatically shortens and simplifies large scale screening of compounds.

According to the present invention, the use of a cumulative marker score is a reliable and efficient tool to evaluate chondrocyte phenotypic stability of a cell population and/or to assess the effect of conditions or compounds on the chondrocyte phenotypic stability of chondrogenic cells. The reliability of the score is determined on the one hand by the correlation between positive scores and the ability to generate phenotypically stable cartilage *in vivo* and the correlation between negative scores and the fact that the cells are not capable of generating phenotypically stable cartilage *in vivo.* An additional desirable requirement of a reliable cumulative marker score is its efficiency, i.e. its ability to efficiency distinguish populations that are and populations that are not capable of generating phenotypically stable cartilage *in vivo.* For instance, where the number of markers is six, and the scoring system varies between +6 and -6, as described above, there is typically a "grey zone" (populations characterized by a cumulative marker score of a range located between +6 and -6), for which the cumulative marker score is not unequivocal and which, when tested would be found to comprise both populations that are and populations that are not capable of forming stable hyaline cartilage. Ideally, the marker score will result in a minimal number of populations in that grey zone, but will allow a maximally efficient classification of populations either as "positive" or as "negative".

More particularly, in the context of determining chondrocyte phenotypic stability of a cell population used for transplantation, both the reliability and the efficiency of the cumulative marker score can be critical, so as to allow a straightforward and correct determination of fate of the cell population. Indeed, the use of a cumulative marker score of a relatively low predictive value for characterizing a population of cells intended for transplantation is of limited value as it does not allow to decide upon implantation or not. For such applications, the use of the Chondrogenic potential score of the present invention as a scoring tool is considered particularly appropriate, in view of its high reliability and efficiency. It is however envisaged that for use in screening, the efficiency of the predictive value may be less critical. As indicated above, a potential purpose of screening assays is to identify pharmaceutical compounds that affect, more particularly improve, the chondrocyte phenotypic stability of a cell population. Thus, in such screening methods, the relative improvement of chondrocyte phenotypic stability can be envisaged to be the critical requirement rather than the ability of the compounds to induce or maintain a perfect chondrocyte phenotypic stability. For example, it is envisaged that upon performing a screening assay, the compounds capable of converting low quality cells (no cartilage after implantation) into intermediate quality cartilage (cells having a score which can not be used to predict unequivocally the result of after implantation), will also be considered of interest.

The cell populations that are used in the methods and assays of the present invention include any cells that are believed to be capable of producing cartilage or that can develop into cartilage producing cells. Typically, the cells are osteochondral cells, such as chondrocytes and their precursor or progenitor cells obtained from articular cartilage, meniscus or synovial fluid. According to one embodiment, the cells used in the context of the present invention are the precursor cells described in WO01/25402. In some embodiments the cell population are precursor cells or stem cells that are derived from other tissues such as blood bone marrow or fat and that can be induced into the osteochondral cell lineage. The assays of the present invention can be used to determine whether these cells have been committed to the osteochondral cell lineage.

The cells used in the screening methods and assays of the invention can be freshly isolated, expanded or passaged for one or more passages. The cells can be present as cell cultures either in culture flasks or present on a matrix or scaffold. The expression of the set of markers by cells on a matrix can be determined by dissolving the matrix and determining the expression by the cells. Cells can be of human or animal origin. Examples of animals that have been used for the study of cartilage are *Xenopus,* zebrafish, chicken, mouse, rat, rabbit, sheep and goat.

The methods and assays of the present invention can be performed with cells, which have a stable chondrocyte phenotype as a result of their origin and/or the cultivation conditions. Such cells are of particular use to assay detrimental effects of a given compound, treatment or condition.

According to an alternative embodiment, the methods and assays of the present invention are performed using cells that do not have a stable chondrocyte phenotype or that have lost the chondrocyte stable phenotype, as a result of their origin and/or cultivation conditions. Examples hereof are chondrocytes that have undergone extensive passaging and stem cells. Also suitable are chondrocytes derived from individuals with an osteochondral defect such as (resulting from) osteoarthritis or rheumatoid arthritis or from individuals which are predisposed to acquire a osteochondral disorder based on to their genetic makeup. Also suitable are cartilage tumour cells or chondrosarcoma cells.

Cells not having chondrocyte phenotypic stability are of particular use in the methods of the present invention to screen for compounds or conditions that improve or restore the stable cartilage phenotype of cells, or that can convert certain cell populations such as stem cells into cells with stable cartilage phenotype.

The present invention envisages the use of the methods and assays of the present invention in a variety of different applications. As detailed above, the methods and assays of the present invention make it possible to test cultivation conditions of cells intended for use in ACT such as cell density, passage number, medium composition, growth in or on two- or three-dimensional substrates, oxygen concentration, pressure, shear stress. In particular the assay allows the testing of the influence of the composition of the medium on chondrocyte phenotypic stability. Factors therein which potentially have an impact on the chondrocyte phenotypic stability include, but are not limited to, the type and batch of serum, different formulations of synthetic serum-free media, and a variety of hormones, growth factors, vitamins, proteins and organic compounds with a alleged effect chondrocyte phenotypic stability.

One application of the methods and assays of the present invention is the screening of compounds or conditions, which differentiate precursor or stem cells into the osteochondral and more particularly in the chondrogenic lineage. Another application is the screening of compounds or conditions which can restore or maintain the chondrocyte phenotypic stability of healthy cells that have been or are being passaged many times, respectively, so as to obtain a sufficient number of cells. Yet another application is the screening of compounds or conditions which can induce chondrocyte phenotypic stability in cells which are obtained from individuals having or predisposed to an osteochondral defect.

In another application, the assay is used to evaluate the impact of matrices, scaffolds, gels or their constituents, which are often used in cell transplantation procedures.

The methods and assays of the present invention are also suitable in classical screenings of peptide libraries, antisense libraries or compound libraries. The compounds making up these libraries include, but are not limited to biologicals and organic or inorganic compounds, which are produced synthetically or obtained from natural sources. Examples hereof are libraries of compounds may be used such as antibody fragment libraries, peptide phage display libraries, peptide libraries (e.g.LOPAP #, Sigma Aldrich), lipid libraries (BioMol), synthetic compound libraries (e.g.LOPAC@, Sigma Aldrich) or natural compound libraries (Specs, TimTec).

The screening methods and assays described herein are of particular use in the identification of lead compounds for medicaments for the treatment of osteochondral disorders. Osteochondral defect envisioned to be of interest in the context of the screening methods include, but are not limited to defects occurring in the joints, such as, but not limited to knee, elbow, ankle, commonly referred to as articular cartilage defects. Cartilage defects are also named after the proximal bone such as defects of the condyles of the femur, of the humerus etc. Frequently occurring cartilage disorders, for which the screening of compounds capable of affecting chondrocyte phenotypic stability are envisaged to be of interest are osteoarthritis, rheumatoid arthritis, articular cartilage injuries, chondromalacia, spondyloarthropathies.

### Brief description of the Figures

The following examples are intended to illustrate the invention without implying any limitation of the invention to the specific embodiments described therein. These examples are illustrated by the following Figures in which,

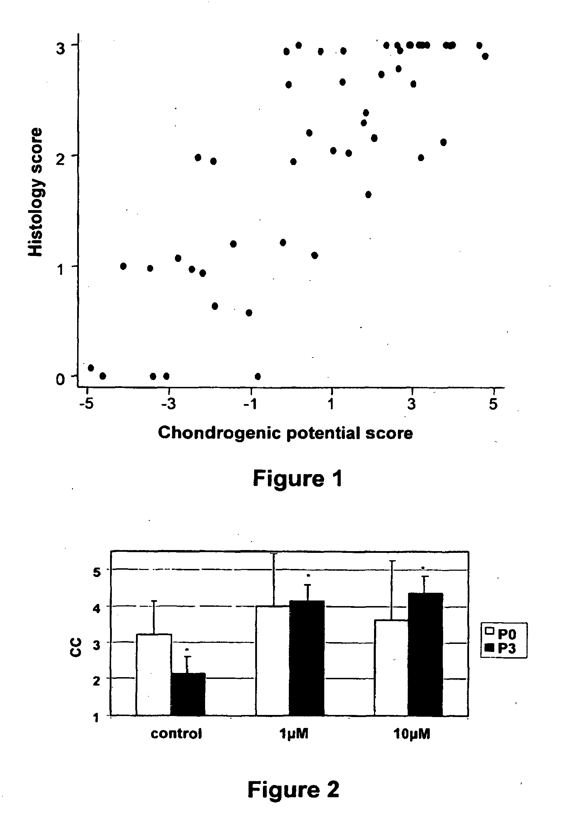

- **Figure 1**: shows a scatter plot of histology score against Chondrogenic potential score (each point has been moved by a small random amount and direction in order to avoid points being superimposed).
- **Figure 2**: shows the effect of compound A on the cumulative marker score (the CC score as determined in Example 1) as determined by Real-Time quantitative PCR on human chondrocytes cultured from P0 until P3 in monolayer. Results represent mean cumulative marker score relative to a control (DMSO). *p<0.05 when compared to DMSO control (n=4).

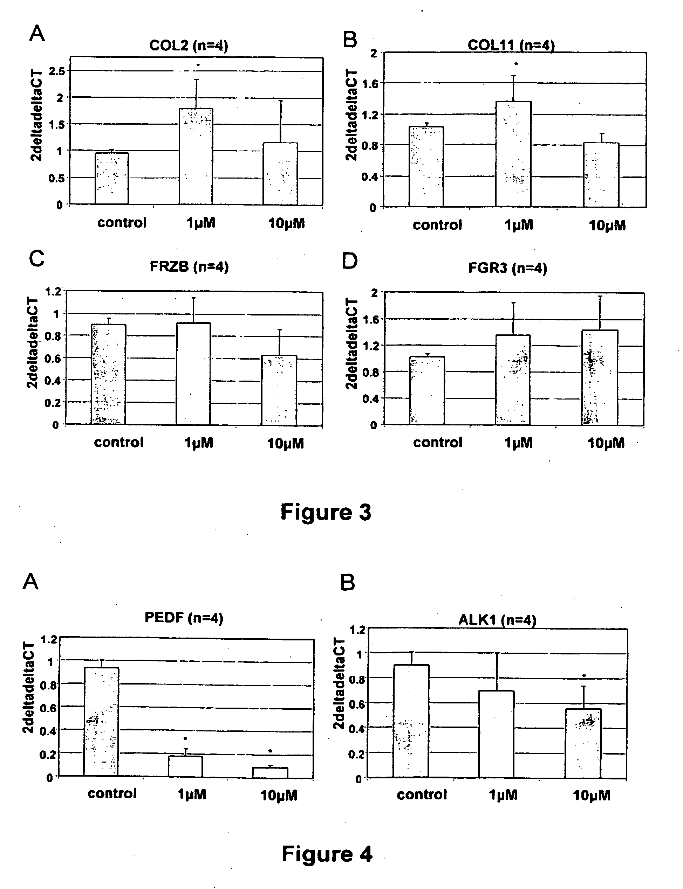

- **Figure 3**: shows the effect of compound A on the expression of molecular markers COL2 (A), COL11(B), FRZB (C) and FGFR3 (D) positively correlated with the chondrogenic potential of human P3 chondrocytes cultured in monolayer as determined by Real-Time quantitative PCR. Results represent target gene expression relative to the DMSO control as determined by the 2^{-deltadeltaCT}. *p<0.05 when compared to DMSO control (n=4).
- **Figure 4**: shows the effect of a compound A on the expression of molecular markers PEDF (A) and ALK1 (B) negatively correlated with the chondrogenic potential of human P3 chondrocytes cultured in monolayer as determined by Real-Time quantitative PCR. Results represent target gene expression relative to DMSO control as determined by the 2^{-deltadeltaCT}. *p<0.05 when compared to DMSO control (n=4).

### EXAMPLES

### Methodology

### Chondrocyte cultivation

Chondrocytes derived from adult human articular cartilage were isolated and expanded *in vitro* using standard conditions as described by Dell'Accio et al. (2001). Arthritis Rheum. 44, 1608-1619).

### In vivo assay

Markers were validated using a set of 48 chondrocyte samples which were obtained from different persons and were freshly isolated or passaged for 1 up to 5 passages. A part of the chondrocytes were used for marker analysis (see example 3) while another part was used in an *in vivo* assay to verify the cartilage forming capacity of each sample. Female NMRI nu/nu mice were injected intramuscularly (posterior compartment of the thigh) with about 5 million human cells. Implants are collected after 2 weeks. [Lipman et al. (1983) Calcif. Tissue Int 35, 767-772; Ostrowski et al. (1975) Somatic Cell Genet. 1, 391-395]. The cartilage implants were dissected from the muscle and histology was performed using hematoxilin-eosin, toluidin blue and Safranin O staining. A histology score ranging from 1 to 3 is attributed to the implant after staining. A histology score of 3 refers to hyaline-like cartilage with highly sulfated proteoglycans, strong staining with toluidine blue and Safranin O. A histology score of 2 refers to well differentiated hyaline-like cartilage, strong staining with toluidine blue, weak staining with Safranin O. A histology score of 1 refers to "fibrocartilage", undifferentiated fibrous tissue, no or weak staining with toluidine blue. A histology score of 0 refers to experiments wherein no implant was retrieved. Cartilage with a histology score of 2 or 3 represents successful implantations. Cartilage with a histology score of 0 or 1 represent failed implantations.

### Example 1: Marker analysis

A fraction of the injected cells was used for gene expression analysis. RNA isolation, reverse transcription and PCR were performed using the methods described in WO0124832. PCR primers for these markers are shown in Table1.

**Table 1: Primers for the amplification of marker genes**

| | Primer | Sequence | SEQ ID. NO: |
|---|---|---|---|
| beta actin | forward | 5'-tgacggggtcacccacactgtgcccatcta-3' | 1 |
| | reverse | 5'-ctagaagcatttgcggtggacgatggaggg-3' | 2 |
| Collagen 11 | forward | 5'-gaactccatctctccctgc-3' | 3 |
| | reverse | 5'-gagactggatttcaaggcaag-3' | 4 |
| Collagen 2 | forward | 5'-ccctgagtggaagagtggag-3' | 5 |
| | reverse | 5'-gaggcgtgaggtcttctgtg-3' | 6 |
| PEDF | forward | 5'-ttcaaggggcagtgggtaac-3' | 7 |
| | reverse | 5'-taaggtgatagtccagcggg-3' | 8 |
| Frzb1 | forward | 5'-tgtaagtctgtgtgcgagcg-3' | 9 |
| | reverse | 5'-gatttagttgcgtgcttgcc-3' | 10 |
| ALK1 | forward | 5'-cgacggaggcaggagaagcag-3' | 11 |
| | reverse | 5'-tgaagtcgcggtgggcaatgg-3' | 12 |
| FGFR3 | forward | 5'-gctgaaagacgatgccactg-3' | 13 |
| | reverse | 5'-aggaccccaaaggaccagac-3' | 14 |

The expression levels of the markers are determined by measuring the intensity of PCR products after electrophoresis. All values are then compared relative to a 600 bp band of a DNA-marker and a value is obtained. The concentration of the individual markers is represented by a numerical value. In the case of a positive marker (COL11, COL2, FGFR3) a very low expression (0-0.1) is represented by -1, a low expression (0.1-1) is represented by a 0 and a high expression is represented by +1. On the contrary In the case of a negative marker (ALK1, PEDF) a very low expression (0-0.1) is represented by +1, a low expression (0.1-1) is represented by a 0 and a high expression is represented by -1. The sum of all these individual markers (defined as Chondrogenic potential score) represent the expression level of the entire set of markers. This number can range from -6 to +6.

The individual data for each marker, its Chondrogenic potential score and histology score are compiled in Table 2.

**Table 2: Overview of histology scores and Chondrogenic potential scores performed on respectively cartilage implants and injected chondrocytes. Sample codes refer to a patient code, a passage number and eventually the confluency of the cells.**

| | | | | **Positive markers** | | | | **Negative markers** | |
|---|---|---|---|---|---|---|---|---|---|
| **Sample** | **Sample code** | **Histology score** | **ChondroCelect score** | **COL11** | **COL2** | **FGFR3** | **FRZB** | **ALK1** | **PEDF** |
| 1 | cs29p0 | 3 | 5 | 3.94 | 8.25 | 3.34 | 4.17 | 0.16 | 0 |
| 2 | cs49p0 80% | 3 | 5 | 0.66 | 1.93 | 1.90 | 2.26 | 0 | 0 |
| 3 | Cs141p3 | 3 | 4 | 1.44 | 6.10 | 3.22 | 2.85 | 0.61 | 0.41 |
| 4 | cs22p0 | 3 | 4 | 5.45 | 10.89 | 6.38 | 5.06 | 0.66 | 0.58 |
| 7 | Cs141p0 | 3 | 3 | 4.95 | 10.95 | 0.34 | 4.42 | 0.39 | 0.35 |
| 8 | Cs145p0 | 3 | 3 | 5.51 | 8.46 | 4.18 | 8.28 | 0.79 | 2.56 |
| 9 | Cs148p0 | 3 | 3 | 2.32 | 11.63 | 3.90 | 6.28 | 0.52 | 1.14 |
| 10 | cs28p0 | 3 | 3 | 2.63 | 9.02 | 4.00 | 4.77 | 0.41 | 1.12 |
| 11 | cs49p0 100% | 3 | 3 | 3.63 | 1.75 | 3.78 | 2.49 | 1.22 | 0.84 |
| 5 | Cs126p1 | 3 | 2 | 2.99 | 3.28 | 2.29 | 1.68 | 1.39 | 1.39 |
| 12 | Cs130p0 | 3 | 2 | 11.96 | 10.77 | 4.87 | 10.89 | 1.23 | 1.83 |
| 13 | Cs134p1 | 3 | 3 | 10.8 | 7.82 | 1.81 | 1.291 | 0.960 | 1.54 |
| 14 | Cs141p2 | 3 | 2 | 7.27 | 13.60 | 0.02 | 5.17 | 0.78 | 0.54 |
| 15 | Cs145p3 | 3 | 2 | 0.10 | 2.53 | 2.35 | 1.10 | 0.22 | 0.31 |
| 16 | Cs146p0 | 3 | 2 | 0.11 | 7.25 | 2.21 | 2.50 | 0.61 | 1.05 |
| 17 | cs49p1 90% | 3 | 2 | 0.51 | 0.18 | 1.53 | 1.21 | 0.20 | 0.43 |
| 6 | Cs136p1 | 3 | 1 | 0.81 | 1.14 | 0.44 | 0.48 | 0.42 | 0.37 |
| 18 | Cs137p3 | 3 | 1 | 0.63 | 3.19 | 1.64 | 0.87 | 1.01 | 0.81 |
| 19 | Cs145p2 | 3 | 1 | 3.19 | 5.08 | 0.29 | 3.97 | 1.73 | 1.60 |
| 20 | Cs146p1fbs | 3 | 1 | 1.47 | 2.00 | 0.79 | 1.61 | 1.57 | 3.82 |
| 21 | Cs130p2 | 3 | 0 | 8.14 | 0.43 | 0.90 | 1.32 | 1.30 | 3.18 |
| 22 | Cs142p1 | 3 | 0 | 0.12 | 3.50 | 1 0.94 | 1.30 | 1.03 | 1.38 |
| 23 | Cs113p1 | 2 | 4 | 3.00 | 3.89 | 3.54 | 1.02 | 0.80 | 0.38 |
| 24 | cs55p1 | 2 | 3 | 1.24 | 3.93 | 1.01 | 1.70 | 0.22 | 3.67 |
| 25 | Cs134p2 | 2 | 2 | 8.18 | 14.54 | 1.99 | 2.45 | 3.09 | 1.33 |
| 26 | Cs127p1 | 2 | 2 | 6.85 | 10.48 | 0.82 | 3.08 | 0.84 | 1.83 |
| 27 | Cs137p4 | 2 | 2 | 0.34 | 1.73 | 0.87 | 1.04 | 0.57 | 0.84 |
| 28 | Cs141p5 | 2 | 2 | 0.37 | 4.23 | 5.22 | 2.28 | 1.06 | 0.30 |
| 29 | cs28p1 | 2 | 2 | 1.20 | 0.53 | 0.90 | 0.65 | 0.52 | 0 |
| 30 | cs43p1 | 2 | 2 | 1.67 | 0 | 1.16 | 0.74 | 0.40 | 0 |
| 31 | cs43p0 | 2 | 1 | 0.64 | 0 | 1.46 | 0.84 | 0.13 | 0 |
| 32 | cs41p1 | 2 | 0 | 0.44 | 0.05 | 1.18 | 0.55 | 0.25 | 0.35 |
| 33 | Cs148p3 | 2 | -1 | 0.31 | 0.87 | 1.63 | 0.88 | 1.36 | 1.63 |
| 34 | Cs139p1 | 2 | -2 | 0.44 | 0.15 | 0.02 | 0.55 | 0.93 | 2.33 |
| 35 | Cs110p3 | 1 | 1 | 1.21 | 1.08 | 2.89 | 0.31 | 2.37 | 2.42 |
| 36 | Cs127p3 | 1 | -1 | 4.22 | 1.05 | 0.01 | 0.89 | 1.42 | 7.72 |
| 37 | cs22p1 | 1 | -1 | 0.45 | 0.33 | 0.42 | 2.41 | 1.23 | 9.22 |
| 38 | cs41p2 | 1 | -1 | 1.26 | 0 | 1.72 | 0.71 | 1.87 | 4.42 |
| 39 | Cs145p5 | 1 | -2 | 0.15 | 0.23 | 0.56 | 0.50 | 2.86 | 2.42 |
| 40 | Cs41p3 | 1 | -2 | 0.95 | 0 | 1.34 | 0.23 | 1.12 | 4.34 |
| 41 | Cs125p5 | 1 | -3 | 3.17 | 0 | 0 | 0.13 | 3.87 | 9.02 |
| 42 | Cs134p5 | 1 | -4 | 0.07 | 0.09 | 0.35 | 0.34 | 1.73 | 1.37 |
| 43 | Cs146p3 | 1 | -5 | 0.07 | 0.01 | 0.06 | 0.15 | 1.65 | 2.67 |
| 44 | cs43p2 | 0 | -1 | 2.56 | 0 | 0.13 | 0.2 | 1.29 | 0.41 |
| 45 | Cs110p5 | 0 | -4 | 0.54 | 0.0 | 0.2 | 0.0 | 1.7 | 7.9 |
| 46 | Cs41p4 | 0 | -3 | 0.14 | 0 | 0.38 | 0.11 | 1.17 | 1.74 |
| 47 | Cs146p5 | 0 | -4 | 0.07 | 0.14 | 0.08 | 0.14 | 2.13 | 3.46 |
| 48 | Cs148p5 | 0 | -4 | 0.05 | 0.02 | 0.16 | 0.14 | 1.57 | 2.34 |

### Example 2: Data analysis

The correlation between the Chondrogenic potential score as determined in Example 1 and the histology score is depicted in Table 3 and in Figure 1. The correlation coefficient between the Chondrogenic potential score and histology score is 0,78 and statistically significant (p<0.0001).

Samples with a Chondrogenic potential score of -3 or lower always result in fibrocartilage or no cartilage at all. Samples with a Chondrogenic potential score of 2 or more will always result in hyaline cartilage. Samples with a Chondrogenic potential score of 1 results in 1 out of 6 cases into hyaline cartilage.

As a yardstick, a sample with a Chondrogenic potential score of 0 or more is considered to be suitable for implantation. Based on the present data set it is possible to predict in 77% (37/48) the outcome of a transplantation with high certainty and in 64% without errors. Based on this data set, a chondrocyte culture is approved for use in an ACI procedure when the Chondrogenic potential score is 0 or more.

In summary, stable hyaline-like cartilage can only be obtained with cells having a Chondrogenic potential score of 0 or higher. Cells that form no cartilage at all had a negative score. Further analysis of individual genes may further help to refine the model.

### a) relationship between Chondrogenic potential score and histology score

The relationship between Chondrogenic potential score and histology score is depicted in Figure 1 and in Table 3.

**Table 3: Frequency table for histology score, with statistical summaries.**

| **Chondro-Celect score** | **histology score** | | | | | | **probability of score of ≥ 2** | |
|---|---|---|---|---|---|---|---|---|
| | 0 | 1 | 2 | 3 | Total | Mean | Observed | Fitted |
| -5 | 0 | 1 | 0 | 0 | 1 | 1.0 | 0/1 (0%) | 0.4% |
| -4 | 2 | 1 | 0 | 0 | 3 | 0.3 | 0/3 (0%) | 1.4% |
| -3 | 2 | 1 | 1 | 0 | 4 | 0.75 | 1/4 25% | 5.0% |
| -2 | 0 | 2 | 0 | 0 | 2 | 1.0 | 0/2 (0%) | 16.2% |
| -1 | 1 | 3 | 1 | 0 | 5 | 1.0 | 1/5 (20%) | 41.5% |
| 0 | 0 | 0 | 1 | 2 | 3 | 2.7 | 3/3 (100%) | 72.4% |
| 1 | 0 | 1 | 1 | 4 | 6 | 2.5 | 5/6 (83%) | 90.6% |
| 2 | 0 | 0 | 6 | 6 | 12 | 2.5 | 12/12 (100%) | 97.3% |
| 3 | 0 | 0 | 1 | 6 | 7 | 2.9 | 7/7 (100%) | 99.2% |
| 4 | 0 | 0 | 1 | 2 | 3 | 2.7 | 3/3 (100%) | 99.8% |
| 5 | 0 | 0 | 0 | 2 | 2 | 3.0 | 2/2 (100%) | 99.9% |
| Total | 5 | 9 | 12 | 22 | 48 | | | |

In addition to the frequency table, Table 3 also shows the mean histology score for each value of the Chondrogenic potential score. This mean value increases significantly (from 1.0 to 2.7) between Chondrogenic potential scores of -1 and 0. This suggests to consider samples with a Chondrogenic potential score of 0 or higher as suitable for ACI.

### b) Data analysis using a categorical scale

An "ordered categorical scale" is a scale in which the categories are numbered purely to show their sequence: the actual numbers assigned provide no other information. For example, the histology scale here takes values of 0, 1, 2, 3. Interpreting this as an ordered categorical scale implies that 1 is better than 0, but says nothing about how much better. For an interval scale, however, the actual numbers are regarded as meaningful, wherein 1 is better than 0 by the same amount that 2 is better than 1, etc. In calculating mean values as in Table 3, the implicit assumption is made that the histology score can be validly treated as an interval scale.

The more points a scale has, the more it is accepted that such scale is an interval scale. The Chondrogenic potential score which is represented by 13 data points is accordingly treated as an interval score.

An additional analysis is presented in the last column of Table 3, which treats the histology score as an ordered categorical scale. Herein the scores are merged into two categories, 0 or 1 (no or low fibrocartilage) and 2 or 3 (hyaline cartilage), the data represent the probability of observing a histology score of 2 or 3. It

In the last two columns of Table 3, the column headed 'observed' shows the actual numbers of samples in this category (and the percentages) for each Chondrogenic potential score. This value also increases between Chondrogenic potential scores -1 and 0, suggesting a threshold value cut-point. For this analysis the Chondrogenic potential score is considered as and ordered categorical scale.

### c) Data analysis using an interval scale

When the Chondrogenic potential score is treated as an interval scale, the observed responses can be smoothed out using logistic analysis. This method predicts the probability of being in the desired category (2 or 3). The fitted values do not go outside the plausible range (0 to 100%). These fitted values are shown in the final column of Table 3, and shows the effect of smoothing. These fitted estimates are assumed to provide a more reliable prediction of the chance of getting a 2 or 3 histology score at any given Chondrogenic potential score than by other methods. Note that in particular for a Chondrogenic potential score of -1, the fitted model is predicting a chance of 41.5% of getting a 2 or 3, and consequently, this raises the question of whether a score of -1 should be accepted.

### Example 3. Impact of individual markers

The impact of individual markers is assessed by recalculating the expression profile of 5 markers instead of 6 markers of the set of markers determining the Chondrogenic potential score in Example 1. The same scoring system was applied, wherein scores now can range from -5 to +5.

Data are presented in Tables 4 to 9.

**Table 4: Frequency table for histology score without COL11 marker**

| Marker score | Histology score | | | | |
|---|---|---|---|---|---|
| | 0 | 1 | 2 | 3 | Total |
| -5 | | | | | 0 |
| -4 | 1 | 2 | | | 3 |
| -3 | 3 | 1 | | | 4 |
| -2 | 1 | 4 | 1 | | 6 |
| -1 | | 1 | 1 | 1 | 3 |
| 0 | | 1 | 1 | 3 | 5 |
| 1 | | | 5 | 5 | 10 |
| 2 | | | 3 | 8 | 11 |
| 3 | | | | 3 | 4 |
| 4 | | | | 1 | 1 |
| 5 | | | | 1 | 1 |
| total | 5 | 9 | 12 | 22 | 48 |

**Table 5: Frequency table for histology score without COL2 marker**

| Marker score | Histology score | | | | |
|---|---|---|---|---|---|
| | 0 | 1 | 2 | 3 | Total |
| -5 | | | | | |
| -4 | 1 | 1 | | | 2 |
| -3 | 2 | 1 | | | 3 |
| -2 | 1 | 3 | 1 | | 5 |
| -1 | | 2 | 1 | 1 | 4 |
| 0 | 1 | 2 | | 5 | 8 |
| 1 | | | 6 | 5 | 11 |
| 2 | | | 3 | 7 | 10 |
| 3 | | | 1 | 2 | 3 |
| 4 | | | | 2 | 2 |
| 5 | | | | | |
| | 5 | 9 | 12 | 22 | 48 |

**Table 6: Frequency table for histology score without PEDF marker**

| Marker score | Histology score | | | | |
|---|---|---|---|---|---|
| | 0 | 1 | 2 | 3 | Total |
| -5 | | | | | |
| -4 | | 1 | | | 1 |
| -3 | 3 | 1 | | | 4 |
| -2 | 1 | 1 | | | 2 |
| -1 | 1 | 2 | 1 | | 4 |
| 0 | | 3 | 2 | | 5 |
| 1 | | | 2 | 4 | 6 |
| 2 | | 1 | 3 | 5 | 9 |
| 3 | | | 2 | 5 | 7 |
| 4 | | | 2 | 8 | 10 |
| 5 | | | | | |
| | 5 | 9 | 12 | 22 | 48 |

**Table 7: Frequency table for histology score without FRZB marker**

| Marker score | Histology score | | | | |
|---|---|---|---|---|---|
| | 0 | 1 | 2 | 3 | Total |
| -5 | | 1 | | | 1 |
| -4 | 2 | 1 | | | 3 |
| -3 | 2 | 1 | | | 3 |
| -2 | | 3 | 1 | | 4 |
| -1 | 1 | 2 | 1 | 2 | 6 |
| 0 | | | 1 | 2 | 3 |
| 1 | | 1 | 5 | 8 | 14 |
| 2 | | | 3 | 6 | 9 |
| 3 | | | 1 | 2 | 3 |
| 4 | | | | 2 | 2 |
| 5 | | | | | |
| | 5 | 9 | 12 | 22 | 48 |

**Table 8: Frequency table for histology score without ALK1 marker**

| Marker score | Histology score | | | | |
|---|---|---|---|---|---|
| | 0 | 1 | 2 | 3 | |
| -5 | | | | | |
| -4 | | 1 | | | 1 |
| -3 | 3 | 1 | | | 4 |
| -2 | 1 | 1 | 1 | | 3 |
| -1 | | 2 | | | 2 |
| 0 | 1 | 3 | 2 | | 6 |
| 1 | | | 1 | 3 | 4 |
| 2 | | 1 | 4 | 7 | 12 |
| 3 | | | 3 | 7 | 10 |
| 4 | | | 1 | 4 | 5 |
| 5 | | | | 1 | 1 |
| | 5 | 9 | 12 | 22 | 48 |

**Table 9: Frequency table for histology score without FGFR3 marker**

| Marker score | Histology score | | | | |
|---|---|---|---|---|---|
| | 0 | 1 | 2 | 3 | Total |
| -5 | | | | | |
| -4 | 2 | 2 | | | 4 |
| -3 | 2 | 1 | | | 3 |
| -2 | | 3 | 1 | | 4 |
| -1 | 1 | 1 | 2 | | 4 |
| 0 | | 2 | 1 | 3 | 6 |
| 1 | | | 3 | 8 | 11 |
| 2 | | | 4 | 5 | 9 |
| 3 | | | 1 | 4 | 5 |
| 4 | | | | 2 | 2 |
| 5 | | | | | |
| | 5 | 9 | 12 | 22 | 48 |

Part A of Table 10 below indicates for each of the data sets presented in Table 4 to 9 above, for a threshold of a marker score, how many of the samples can be unequivocally classified as giving low quality (histology score of 0 or 1 corresponding to '-' in Table 10) or giving high quality cartilage (histology score of 2 or 3, indicated by '+' in Table 10). Samples for which there is no correlation between the marker score and histology score are classified as '?' samples. These data indicate that when a set of 5 markers is used wherein COL11, COL2 or COL11 or omitted, similar or even better results are obtained (i.e. fewer samples designated as '?').

In part B of Table 10 the cut off values are less restricted. When for one marker score within the indicated range, at least 5 out of 6 samples show a correlation between marker score and cartilage quality, the range of the marker score is assumed to be predictive. Using this approach omission of COL11 results in improved results while omission of any other marker gives worse results, especially when Col2 or FGFR3 are omitted from the markers set of Example 1.

**Table 10: Predictive values of a marker set containing 5 markers.**

| **A** | 6 markers | -COL11 | -COL2 | -PEDF | -FRZB | -ALK1 | -FGFR3 |
|---|---|---|---|---|---|---|---|
| - | 7 | 7 | 5 | 7 | 7 | 5 | 7 |
| ? | 17 | 14 | 17 | 24 | 27 | 27 | 14 |
| + | 24 | 27 | 26 | 17 | 14 | 16 | 27 |
| | | | | | | | |

| **B** | 6 markers | -COL11 | -COL2 | -PEDF | -FRZB | -ALK1 | -FGFR3 |
|---|---|---|---|---|---|---|---|
| - | 7 | 13 | 10 | 7 | 7 | 5 | 7 |
| ? | 8 | 3 | 12 | 9 | 10 | 11 | 14 |
| + | 33 | 32 | 26 | 32 | 27 | 32 | 27 |

Based on the present set of data and their interpretation, it appears that the predictive power of the expression profile of a set of markers for the *in vivo* generation of cartilage can optionally also be obtained without COL11 regardless of the interpretation. On the other hand omission of either ALK1 or FRZB results in a decreased predictive power of the markers, regardless of the interpretation of the present data set.

### Example 4. Use of the marker score in screening assays

The evaluation of chondrocyte phenotypic stability using a cumulative marker score was tested in screening assays to identify compounds capable of affecting the ability of cells to produce stable cartilage *in vivo* and to determine the nature of the effect of such compounds on the cells.

Screening assays were performed in 96 well plates wherein between 10 000 and 100 000 chondrocytes obtained from a cartilage biopsy were seeded (freshly isolated from passage 0 to P5). Test compounds were added in varying concentrations and for varying time periods. After incubation, chondrocytes were harvested, RNA was isolated and treated with reverse transcriptase. Quantitative PCR was performing according to the instructions of the TaqMan procedure, and the Chondrogenic potential score of the cells was determined as described in Example 1 (CC score).

### 1. Testing for molecules that counteract the dedifferentiation of chondrocytes in monolayer culture

Compound A was a candidate compound which was predicted to have a chondroprotective effect and to have a putative stabilizing effects on the chondrocyte phenotype. The determination of a cumulative marker score, more particularly the chondrogenic potential score of Example 1, was used to determine the effect of this compound on the chondrocyte phenotypic stability of a chondrocyte monolayer culture.

### a) Chondrocyte monolayer cultures

Chondrocytes were grown in monolayer culture at 4x10⁴ cells/ml in T25 flasks. Compound A was added to the cells directly after plating. A total final volume of 5 ml complete media (DMEM + 10% FBS) was added. Three flasks were used for each condition (1 or 10µM compound A, DMSO control). Media and compounds were replaced once a week. At confluency, chondrocytes at passage 0 (P0) were trypsinized, counted and 2x10⁵ cells were replated for P1 culture. The remaining cells were put in lysis media for RNA extraction. The same procedure was performed for P1, P2 and P3 chondrocyte cultures. TaqMan RT-PCR was used to determine the expression of chondrogenic markers.

### b) RNA isolation, cDNA generation

Chondrocytes obtained from monolayer culture were washed with PBS and lyzed using RLT buffer containing 1% mercaptoethanol (Qiagen). Lysed cells were stored at -80°C until used. mRNA was purified using the RNeasy microkit (Qiagen) according to the manufacturer instructions. cDNA was synthesised using random hexamers following the manufacturer instructions (Invitrogen).

### c) TaqMan real time polymerase chain reaction (RT-PCR)

Real-Time quantitative PCR (RT-PCR) was performed using the ABI prism 7700 sequence detector system. The Mastermix qPCR kit (Eurogentec) was used for RT-PCR according to the manufacturer's instructions. The primers and probes used for the TaqMan analysis were ordered from Applied Biosystems or Eurogentec. Real-Time PCR data were calculated using the 2^{-deltadeltaCT} (2DDCT) method which is defined as the amount of target gene normalised to an endogenous reference (β-actin) and relative to a calibrator, which in this case are the expression levels of selected genes under control conditions (DMSO).

### d) Results

Compound A had an effect on the expression of several molecular markers indicative for the chondrogenic capacity, phenotypic stability and homeostasis of chondrocytes and was able to counteract the decrease in the chondrogenic potential score during expansion of chondrocytes in monolayer culture (see Figure 2) Thus both at 1µM and 10µM concentrations, the chondrogenic potential scores for chondrocytes at P0 and P3 were comparable or even increased compared to chondrocytes grown in the absence of the compound. In the latter case a drop in cumulative marker score was observed for P3 versus P0 chondrocytes.

Compound A had a positive effect on the gene expression of several of the extracellular matrix components of chondrocytes. At lower (1µM) but not higher (10µM) concentrations, compound A induced the expression of both Col2 and Col11 gene transcripts (Figure 3). The compound also increases the expression of the FGFR-3 marker although the increase was not statistically significant. Moreover, compound A strongly inhibited the expression of PDEF at both concentrations tested (Figure 4). At 10µM, the addition of the compound also resulted in a reduced expression of ALK1 (Figure 4). Both markers, when upregulated, correlate negatively with the chondrogenic capacity of the cells indicating that compound A has a positive and stimulatory effect on the chondrogenic potential of the chondrocytes cells and thus on chondrogenesis.

### 2. Screening molecules for their ability to mediate the re-differentiation of de-differentiated chondrocytes in 3D culture.

Fourteen compounds were screened for their ability to enhance the re-differentiation of dedifferentiated chondrocytes in alginate cultures. The effect of the molecules on the expression of the different molecular markers and on the cumulative marker score (chondrogenic potential score as determined in Example 1) were determined, in order to assess the ability of the compounds to affect the recovery of the chondrogenic phenotype of the cells in the 3D cultures.

### a) Redifferentiation of chondrocytes in alginate culture

After expansion in monolayer culture in complete media, human chondrocytes at passage 3 (P3) taken from the knee of 5 OA patients (age 50-65) were released by trypsin treatment, counted and tested for viability by the trypan exclusion test. Chondrocytes were suspended in 2% alginate and equal amount of HBSS was added. The cell suspension was sucked into a 10ml syringe needle and transferred dropwise by a 24 Gauge into a calcium chloride solution (5 beads per well, total cell number 250000/well). Beads were washed with NaCl and new complete media (DMEM + 10% FBS) was added. After 4 days of culture the supernatant was removed and new media was added with the compounds at a concentration of 10µM. Four days after addition of the compounds, the supernatant was removed and the cells were released from their beads for lysis. mRNA and cDNA were generated and TaqMan RT-PCR were used to measure the expression of COL2, COL11, FGR3, FRZB, ALK1, PEDF as described in Example 1.

### b) Results

The relative effects of the compounds on the up or down regulation of marker gene expression in chondrocytes grown in alginate is summarized in Table 11. The effects are relative to chondrocytes grown in the absence of molecules (but presence of vehicle DMSO). Results represent the mean expression (mean of 5 OA patients) relative to controls (chondrocytes plus DMSO). NS = not significant; - = inhibits; + = enhances; # = p<0.05 significant when compared to control DMSO. Compounds were used at 10 µM.

Several molecules were able to affect the expression of various marker genes in a positive or negative way. Only two compounds (Cpd 8 and Cpd 14) had a positive effect on the cumulative marker score (CC score) indicating that these compounds could enhance/mediate re-differentiation of the chondrocytes. However, only Cpd 14 had the desired effect on each of the individual markers, with no effect on BMP2 or ALK1 gene expression. This compound could be useful in stimulating chondrocyte de-differentiation *ex vivo* before re-implantation and could thus tip the balance towards formation of articular hyaline cartilage *in vivo.*

**Table 11. Effect of test compounds on the expression of phenotypic modulation of dedifferentiated chondrocytes grown in alginate cultures.**

| *compounds* | COL2 | FGR3 | BMP2 | COL11 | *ALK1* | *PEDF* | *CC score* |
|---|---|---|---|---|---|---|---|
| Cpd 1 | NS | -^{#} | NS | NS | NS | -^{#} | NS |
| Cpd 2 | -^{#} | NS | -^{#} | NS | NS | -^{#} | NS |
| Cpd 3 | -^{#} | -^{#} | NS | -^{#} | NS | -^{#} | NS |
| Cpd 4 | -^{#} | NS | NS | NS | NS | NS | NS |
| Cpd 5 | -^{#} | -^{#} | -^{#} | NS | NS | -^{#} | -^{#} |
| Cpd 6 | -^{#} | -^{#} | -^{#} | NS | NS | -^{#} | -^{#} |
| Cpd 7 | NS | -^{#} | NS | NS | NS | NS | NS |
| Cpd 8 | NS | NS | NS | -^{#} | -^{#} | NS | +^{#} |
| Cpd 9 | NS | NS | NS | NS | NS | NS | NS |
| Cpd 10 | -^{#} | NS | NS | NS | NS | NS | NS |
| Cpd 11 | -^{#} | -^{#} | +^{#} | NS | NS | -^{#} | -^{#} |
| Cpd 12 | -^{#} | NS | +^{#} | NS | -^{#} | -^{#} | NS |
| Cpd 13 | -^{#} | NS | NS | NS | NS | -^{#} | -^{#} |
| Cpd 14 | +^{#} | +^{#} | NS | +^{#} | NS | -^{#} | +^{#} |

### Example 5. Testing of the effect of chondroprotective compounds in vivo

For those compounds of Example 4 significantly affecting the Chondrogenic potential score, the effect of the compounds on the chondrocyte phenotypic stability of the cells is also tested *in vivo.* To this end, cell populations are contacted with either the test compound or buffer and, after incubation, are injected into the nude mice model (see above). The ability of each of the cell populations to produce stable hyaline cartilage is evaluated. It is established that the effect of the compound on the chondrogenic potential score correlates with the effect on the chondrogenic potential of the cell population.

## Claims

1. An in vitro method for determining the ability of a cell population to produce stable hyaline cartilage in vivo, which comprises determining the expression by said population of a set of at least three marker genes comprising FRZB (Frizzled-Related Protein 1), ALK1 (Activin A Receptor, Type II-Like Kinase 1) and one or more markers selected from the group consisting of PEDF (Pigment Epithelium-Derived Factor), COL 11 (Collagen, Type XI A1), COL2 (Collagen, Type II, Alpha 1), FGFR3 (Fibroblast Growth Factor Receptor 3), OPN (Osteopontin), BMP-2 (Bone Morphogenetic Protein 2) and RASF-PLA (Phospholipase A2, Group IIA).

2. The method of claim 1, whereby the cell population is contacted with a compound or condition.

3. The method according to claim 2, wherein said compound is selected from the group consisting of growth factors, mitogens, additives, and small chemical molecules, or wherein said condition is selected from the group consisting of a specific cultivation medium, cultivation temperature, cultivation time, cultivation density and the combination of a cell population of claim 1 with another cell type.

4. The method of claim 2 or 3, comprising the steps of:
- contacting the population of cells with said compound or condition, and
- determining the expression level by said population of cells of a set of at least three marker genes comprising FRZB, ALK1 and one or more marker genes selected from the group consisting of PEDF, COL 11, COL2, FGFR3, OPN, BMP-2 and RASF-PLA.

5. The method of claim 4, further comprising the steps of:
- prior to said contacting step, determining the expression level by said population of cells of said set of at least three marker genes, and
- after said contacting step, determining whether the compound or condition is capable of affecting the expression of one or more of said set of at least three marker genes.

6. The method of any one of claims 1 to 5, wherein the set of at least three markers are a set of at least 4, 5 or 6 marker genes comprising FRZB, ALK1 and comprising respectively 2, 3 and 4 marker genes selected from the group consisting of PEDF, COL 11, COL2, and FGFR3.

7. The method of any one of claims 1 to 6, wherein the set of at least three marker genes is a set of at least six marker genes comprising FRZB, ALK1, PEDF, COL 11, COL2 and FGFR3.

8. The method according to claim 4 or 5, which further comprises determining, based on the effect of the presence of the compound on the expression of the set of at least three marker genes, the ability of the compound to affect the ability of the cell population to produce stable hyaline cartilage in vivo.

9. The method according to claim 8, wherein a compound capable of increasing the expression of one or more positive marker genes selected from the group consisting of FRZB, COL 11, COL2, FGFR3, OPN, BMP-2 and RASF-PLA are identified as positively affecting cartilage formation and compounds capable of increasing the expression of either PEDF and/or ALK-1 are identified as negatively affecting cartilage formation.

10. The method according to claim 9, wherein the ability of a compound or condition to affect cartilage formation is determined based on the cumulative effect of the compound or condition on the expression of the positive marker genes selected from the group consisting of FRZB, COL 11, COL2, FGFR3, OPN, BMP-2, RASF-PLA and the negative marker genes PEDF and/or ALK- 1.

11. The method according to any one of claims 1 to10, wherein the population of cells is obtained from a joint.

12. Use of a set of markers for identifying a compound that is capable of affecting the ability of cells to produce cartilage formation in vitro, **characterised in that** the set of markers comprises a set of at least three marker genes comprising FRZB, ALK1 and one or more markers selected from the group consisting of PEDF, COL 11, COL2, FGFR3, OPN, BMP-2 and RASF-PLA.

13. The use according to claim 12, wherein the set of markers comprises at least six markers comprising FRZB, ALK 1, PEDF, COL 11, COL2 and FGFR3.

14. A kit comprising probes for detecting the expression of a set of genes in cartilage forming cells wherein the set of genes consists of FRZB, ALK1, PEDF, COL 11, COL2 and FGFR3.

15. The kit according to claim 14, wherein the probes for detection of at least one of the marker genes are selected from the group consisting of oligonucleotides which hybridise with mRNA, sets of PCR primers and antibodies.

16. A device for detecting the expression of a set of genes in cartilage forming cells, said device comprising the following:
- a unit for detecting the expression of the marker genes,
- and probes for a set of marker genes consisting pf FRZB, ALK1, PEDF, COL 11, COL2 and FGFR3.

## Patentansprüche

1. *In-Vitro*-Verfahren zum Bestimmen der Fähigkeit einer Zellpopulation zur Bildung von stabilem hyalinem Knorpel *in vivo,* welches das Bestimmen der Expression eines Satzes von mindestens drei Markergenen durch die Population umfasst, die FRZB (Frizzled-Related-Protein 1), ALK1 (Activin-A-Rezeptor, Typ-II-artige Kinase 1) und einen oder mehrere Marker umfassen, der/die aus der Gruppe ausgewählt ist/sind, die aus PEDF (Pigmentepithel-assoziierter Faktor), COL 11 (Collagen, Typ XI A1), COL2 (Collagen, Typ II, Alpha 1), FGFR3 (Fibroblastenwachstumsfaktorrezeptor 3), OPN (Osteopontin), BMP-2 (Bone Morphogenetic Protein 2 bzw. morphogenetisches Knochenprotein 2) und RASF-PLA (Phospholipase A2, Gruppe IIA) besteht.

2. Verfahren nach Anspruch 1, wobei die Zellpopulation mit einer Verbindung oder einem Zustand in Kontakt gebracht wird.

3. Verfahren nach Anspruch 2, wobei die Verbindung aus der Gruppe ausgewählt ist, die aus Wachstumsfaktoren, Mitogenen, Zusatzstoffen und kleinen chemischen Molekülen besteht, oder wobei der Zustand aus der Gruppe ausgewählt ist, die aus einem bestimmten Kultivierungsmedium, einer bestimmten Kultivierungstemperatur, einer bestimmten Kultivierungsdauer, einer bestimmten Kultivierungsdichte und der Kombination aus einer Zellpopulation von Anspruch 1 mit einem anderen Zelltyp besteht.

4. Verfahren nach Anspruch 2 oder 3, das die folgenden Schritte umfasst:
- In Kontakt bringen der Population von Zellen mit der Verbindung oder dem Zustand, und
- Bestimmen des Expressionsgrades durch die Population von Zellen eines Satzes von mindestens drei Markergenen, die FRZB, ALK1 und ein oder mehrere Markergen(e) umfassen, das(die) aus der Gruppe ausgewählt ist(sind), die aus PEDF, COL 11, COL2, FGFR3, OPN, BMP-2 und RASF-PLA besteht.

5. Verfahren nach Anspruch 4, das ferner die folgenden Schritte umfasst:
- Bestimmen des Expressionsgrades durch die Population von Zellen des Satzes von mindestens drei Markergenen vor dem Schritt des In-Kontakt-Bringens, und
- Bestimmen, ob die Verbindung oder der Zustand zum Beeinflussen der Expression von einem oder mehreren aus dem Satz von mindestens drei Markergenen in der Lage ist, nach dem Schritt des In-Kontakt-Bringens.

6. Verfahren nach einem der Ansprüche 1 bis 5, wobei es sich bei dem Satz von mindestens drei Markern um einen Satz von mindestens 4, 5 oder 6 Markergenen handelt, die FRZB, ALK1 umfassen und 2, 3 bzw. 4 Markergene umfassen, die aus der Gruppe ausgewählt sind, die aus PEDF, COL 11, COL2 und FGFR3 besteht.

7. Verfahren nach einem der Ansprüche 1 bis 6, wobei es sich bei dem Satz von mindestens drei Markern um einen Satz von mindestens sechs Markergenen handelt, die FRZB, ALK1, PEDF, COL 11, COL2 und FGFR3 umfassen.

8. Verfahren nach Anspruch 4 oder 5, welches ferner das Bestimmen der Fähigkeit der Verbindung zum Beeinflussen der Fähigkeit der Zellpopulation zur Bildung von stabilem hyalinem Knorpel *in vivo* auf der Grundlage der Auswirkung des Vorhandenseins der Verbindung auf die Expression des Satzes von mindestens drei Markergenen umfasst.

9. Verfahren nach Anspruch 8, wobei eine Verbindung, die zum Erhöhen der Expression von einem oder mehreren positiven Markergen(en), das(die) aus der Gruppe ausgewählt ist(sind), die aus FRZB, COL 11, COL2, FGFR3, OPN, BMP-2 und RASF-PLA besteht, als die Knorpelbildung positiv beeinflussend identifiziert wird und Verbindungen, die zum Erhöhen der Expression von entweder PEDF und/oder ALK-1 in der Lage sind, als die Knorpelbildung negativ beeinflussend identifiziert werden.

10. Verfahren nach Anspruch 9, wobei die Fähigkeit einer Verbindung oder eines Zustands zur Beeinflussung von Knorpelbildung auf der Grundlage der kumulativen Auswirkung der Verbindung oder des Zustands auf die Expression der positiven Markergene, die aus der Gruppe ausgewählt sind, die aus FRZB, COL 11, COL2, FGFR3, OPN, BMP-2 und RASF-PLA besteht, und der negativen Markergene PEDF und/oder ALK-1 bestimmt wird.

11. Verfahren nach einem der Ansprüche 1 bis 10, wobei die Population von Zellen aus einem Gelenk erhalten wird.

12. Verwendung eines Satzes von Markern zum Identifizieren einer Verbindung, die zum Beeinflussen der Fähigkeit von Zellen zum Hervorbringen von Knorpelbildung *in vitro* in der Lage ist, **dadurch gekennzeichnet, dass** der Satz von Markern einen Satz von mindestens drei Markergenen umfasst, die FRZB, ALK1 umfassen, und einen oder mehrere Marker, die aus der Gruppe ausgewählt sind, die aus PEDF, COL 11, COL2, FGFR3, OPN, BMP-2 und RASF-PLA besteht.

13. Verwendung nach Anspruch 12, wobei der Satz von Markern mindestens sechs Marker umfasst, die FRZB, ALK1, PEDF, COL 11, COL2 und FGFR3 umfassen.

14. Kit, welches Sonden zum Detektieren der Expression eines Satzes von Genen in knorpelbildenden Zellen umfasst, wobei der Satz von Genen aus FRZB, ALK1, PEDF, COL 11, COL2 und FGFR3 besteht.

15. Kit nach Anspruch 14, wobei die Sonden zum Detektieren mindestens eines der Markergene aus der Gruppe ausgewählt sind, die aus Oligonukleotiden besteht, welche mit mRNA, Sätzen von PCR-Primern und Antikörpern hybridisieren.

16. Vorrichtung zum Detektieren der Expression eines Satzes von Genen in knorpelbildenden Zellen, wobei die Vorrichtung Folgendes umfasst:
- eine Einheit zum Detektieren der Expression der Markergene,
- und Sonden für einen Satz von Markergenen, die aus FRZB, ALK1, PEDF, COL 11, COL2 und FGFR3 bestehen.

## Revendications

1. Procédé in vitro pour déterminer la capacité d'une population de cellules à produire un cartilage hyalin stable in vivo, qui comprend la détermination de l'expression par ladite population d'un ensemble d'au moins trois gènes marqueurs comprenant FRZB (protéine 1 apparentée à Frizzled), ALK1 (Récepteur d'Activine A, Kinase 1 semblable au Type II) et un ou plusieurs marqueurs sélectionnés à partir du groupe constitué par PEDF (Facteur Dérivé de l'Épithélium Pigmentaire), COL 11 (Collagène, Type XI A1), COL2 (Collagène, Type II, Alpha 1), FGFR3 (Récepteur 3 de Facteur de croissance Fibroblastique 3), OPN (Ostéopontine), BMP-2 (Protéine 2 morphogénétique osseuse) et RASF-PLA (Phospholipase A2, Groupe IIA).

2. Procédé selon la revendication 1, dans lequel la population de cellules est mise en contact avec un composé ou une condition.

3. Procédé selon la revendication 2, dans lequel ledit composé est sélectionné à partir du groupe constitué par des facteurs de croissance, des mitogènes, des additifs et des petites molécules chimiques, ou dans lequel ladite condition est sélectionnée à partir du groupe constitué par un milieu de culture, une température de culture, un temps de culture, une densité de culture spécifiques et la combinaison d'une population de cellules de la revendication 1 avec un autre type de cellule.

4. Procédé selon la revendication 2 ou 3, comprenant les étapes de :
- mise en contact de la population de cellules avec ledit composé ou ladite condition, et
- détermination du niveau d'expression par ladite population de cellules d'un ensemble d'au moins trois gènes marqueurs comprenant FRZB, ALK1 et un ou plusieurs gènes marqueurs sélectionnés à partir du groupe constitué par PEDF, COL 11, COL2, FGFR3, OPN, BMP-2 et RASF-PLA.

5. Procédé selon la revendication 4, comprenant en outre les étapes de :
- avant ladite étape de mise en contact, détermination du niveau d'expression par ladite population de cellules dudit ensemble d'au moins trois gènes marqueurs, et
- après ladite étape de mise en contact, détermination si le composé ou la condition est susceptible d'affecter l'expression d'un ou plusieurs marqueurs dudit ensemble d'au moins trois gènes marqueurs.

6. Procédé selon l'une quelconque des revendications 1 à 5, dans lequel l'ensemble d'au moins trois marqueurs est un ensemble d'au moins 4, 5 ou 6 gènes marqueurs comprenant FRZB, ALK1 et comprenant respectivement 2, 3 et 4 gènes marqueurs sélectionnés à partir du groupe constitué par PEDF, COL 11, COL2 et FGFR3.

7. Procédé selon l'une quelconque des revendications 1 à 6, dans lequel l'ensemble d'au moins trois gènes marqueurs est un ensemble d'au moins six gènes marqueurs comprenant FRZB, ALK1, PEDF, COL 11, COL2 et FGFR3.

8. Procédé selon la revendication 4 ou 5, qui comprend en outre la détermination, en se basant sur l'effet de la présence du composé sur l'expression de l'ensemble d'au moins trois gènes marqueurs, de la capacité du composé à affecter la capacité de la population de cellules à produire le cartilage hyalin stable in vivo.

9. Procédé selon la revendication 8, dans lequel un composé susceptible d'augmenter l'expression d'un ou plusieurs gènes marqueurs positifs sélectionnés à partir du groupe constitué par FRZB, COL 11, COL2, FGFR3, OPN, BMP-2 et RASF-PLA est identifié comme affectant positivement la formation de cartilage et des composés susceptibles d'augmenter l'expression de l'un ou l'autre de PEDF et/ou d'ALK-1 sont identifiés comme affectant négativement la formation de cartilage.

10. Procédé selon la revendication 9, dans lequel la capacité d'un composé ou d'une condition à affecter la formation de cartilage est déterminée en se basant sur l'effet cumulatif du composé ou de la condition sur l'expression des gènes marqueurs positifs sélectionnés à partir du groupe constitué par FRZB, COL 11, COL2, FGFR3, OPN, BMP-2, RASF-PLA et les gènes marqueurs négatifs PEDF et/ou ALK-1.

11. Procédé selon l'une quelconque des revendications 1 à 10, dans lequel la population de cellules est obtenue à partir d'une articulation.

12. Utilisation d'un ensemble de marqueurs pour identifier un composé qui est susceptible d'affecter la capacité de cellules à produire une formation de cartilage in vitro, **caractérisée en ce que** l'ensemble de marqueurs comprend un ensemble d'au moins trois gènes marqueurs comprenant FRZB, ALK1 et un ou plusieurs marqueurs sélectionnés à partir du groupe constitué par PEDF, COL 11, COL2, FGFR3, OPN, BMP-2 et RASF-PLA.

13. Utilisation selon la revendication 12, dans lequel l'ensemble de marqueurs comprend au moins six marqueurs comprenant FRZB, ALK 1, PEDF, COL 11, COL2 et FGFR3.

14. Kit comprenant des sondes pour détecter l'expression d'un ensemble de gènes dans des cellules de formation de cartilage, dans lequel l'ensemble de gènes est constitué par FRZB, ALK1, PEDF, COL 11, COL2 et FGFR3.

15. Kit selon la revendication 14, dans lequel les sondes pour la détection d'au moins un des gènes marqueurs sont sélectionnées à partir du groupe constitué par des oligonucléotides qui s'hybrident avec de l'ARNm, des ensembles d'amorces PCR et des anticorps.

16. Dispositif pour détecter l'expression d'un ensemble de gènes dans des cellules de formation de cartilage, ledit dispositif comprenant ce qui suit :
- une unité pour détecter l'expression des gènes marqueurs,
- et des sondes pour un ensemble de gènes marqueurs constitué par FRZB, ALK1, PEDF, COL 11, COL2 et FGFR3.
